# EUROPÄISCHE PATENTSCHRIFT

(11) **EP 1 883 400 B1**
(45) Veröffentlichungstag und Bekanntmachung des Hinweises auf die Patenterteilung: **08.10.2008**
(21) Anmeldenummer: 06742966.2
(22) Anmeldetag: 17.05.2006
(51) Int. Cl.: A61K 9/48

(54) **KAPSELN FÜR INHALATOREN**
INHALATOR CAPSULES
CAPSULES POUR INHALATEURS

(30) Priorität: 18.05.2005 DE 102005022862
(43) Veröffentlichungstag der Anmeldung: 06.02.2008
(73) Patentinhaber: Airsec S.A.S., 94603 Choisy Le Roi (FR); Boehringer Ingelheim Pharma GmbH & Co. KG, 55216 Ingelheim am Rhein (DE)
(72) Erfinder: LANCESSEUR, Didier, F-92100 Boulogne Billancourt (FR); HOCHRAINER, Dieter, 57392 Schmallenberg (DE); SCHIEWE, Jörg, 55129 Mainz (DE); ZIERENBERG, Bernd, 55411 Bingen (DE)
(74) Vertreter: Westendorp, Michael Oliver
(86) Internationale Anmeldenummer: PCT/EP2006/004684
(87) Internationale Veröffentlichungsnummer: WO 2006/122790

(56) Entgegenhaltungen:
- WO-A-00/07572
- WO-A-87/01034
- WO-A-20/04062716

## Beschreibung

Die Erfindung betrifft eine Kapsel als Primärverpackung, insbesondere für pharmazeutische Inhalationsformulierungen. Die erfindungsgemäße Kapsel ist integraler Bestandteil eines einsatzfähigen Pulverinhalators.

### Stand der Technik

Die medizinische, auf die pulmonale Inhalation ausgerichtete Aerosoltherapie spielt eine wichtige Rolle in der Behandlung von zahlreichen Lungenkrankheiten. Neben Vemeblem von wirkstoffhaltigen Flüssigkeiten werden besonders Pulverinhalatoren zur Applikation von pulverförmigen Wirkstoffformulierungen eingesetzt.

Auf dem Gebiet der Pulverinhalatoren sind Einzeldosen und Mehrdosen-Geräte bekannt. In Einzeldosen-Pulverinhalatoren kann die Dosierung in Form von zumeist zylinderartigen Kapseln vorgenommen werden, die eine Pulverformulierung enthalten. Pulverformulierungen enthalten den Wirkstoff in mikronisierter Form (mit einer Teilchengröße von ca. 1 - 5 µm), und meist ein oder mehrere Hilfsstoffe. Wird eine Kapsel als Behältnis verwendet, dann wird diese in den Pulverinhalatoren vor dem Inhalationsmanöver durch Anstechen, Quetschen oder Scheren geöffnet, damit das Pulver durch den Atemzug des Patienten aus der Kapsel gefördert werden kann und ein luftgetragenes Aerosol erzeugt wird, das der Patient einatmet. Je nach Gerät können ein oder mehrere Kapseln gemeinsam in dem Gerät bevorratet werden oder jede Kapsel wird bei Gebrauch einzeln in das Gerät eingesetzt.

Die bevorzugt in einem Pulverinhalator verwendeten Kapseln bestehen aus zwei oder mehreren Teilen und besitzen vorzugsweise die Größe 3. Ein solcher Pulverinhalator ist beispielsweise ein Inhalator der Marke HandiHaler^{®}, wie er z.B. in der EP 1342483 offenbart wird.

Die Art und Weise, wie die zur Inhalation vorgesehene Pulverformulierung von der Kapsel verpackt vorliegt, ist für die Produktqualität und damit die Eignung für die inhalative Anwendung entscheidend. In der Regel kommt dabei das Inhalationspulver direkt mit dem Kapselmaterial in Berührung, so dass die Qualitätskriterien für Primärverpackung zu beachten sind. Gegebenenfalls kann dabei die Primärverpackung von einem zweiten äußeren Schutz umgeben sein, dem Sekundärpackmittel, das vor der Verwendung entfernt werden muss. Das Sekundärpackmittel umschließt dabei in der Regel das Primärpackmittel vollständig. Sekundärpackmittel werden insbesondere dann verwendet, wenn das Primärpackmittel keinen zeitlich unbegrenzten oder ausreichenden Schutz vor z.B. Feuchtigkeit oder anderen externen Einflüssen bietet. Derartige Sekundärpackmitteln können z.B. Folienbehältnisse aus einer Aluminiumfolie sein (Blister u.ä.).

In solchen Fällen wird in der Regel das Sekundärpackmittel zunächst entfernt, bevor die geschlossene Kapsel in den Pulverinhalator eingebracht wird. Dort wird die Kapsel dann durch entsprechende Mittel des Pulverinhalators geöffnet.

Die Auswahl eines geeigneten Materials für die Kapseln wird durch zwei Faktoren bestimmt: Zum einen muss das Material eine gewisse Schutzfunktion erfüllen können. Zum anderen muss das Material derart sein, dass der Kapsel die zur Verwendung in dem Pulverinhalator notwendige Form gegeben werden kann und die ihr zugedachte Funktion erfüllen kann. Im Fall des HandiHalers^{®} muss die Kapsel derart sein, dass das Pulver über den durch das Einatmen des Patienten erzeugten Bernoullieffekt ausgebracht werden kann.

Kapseln für Pulverinhalatoren bestehen normalerweise aus Hartgelatine, Cellulosederivaten, Stärke, Stärkederivaten, Chitosan usw. aber es sind auch Kapseln aus synthetischen Kunststoffen, wie Polyethylen, Polycarbonat, Polyester, Polypropylen oder Polyethylenterephthalat u.a. bekannt.

Die bisher verwendeten Materialien haben den Nachteil, dass sie in beide Richtungen für Luftfeuchtigkeit durchlässig sind. Damit besteht ein Bedarf, die Fähigkeit der Kapseln, das Inhalationspulver stabil aufzubewahren, zu erhöhen. Zudem sind Materialien wie Gelatine empfindlich gegenüber Feuchtigkeit und können bei zu großer Trockenheit brechen. Unter feuchten Bedingungen werden sie andererseits klebrig, wodurch eine effiziente Befüllung und/oder Verwendung der Kapseln, beispielsweise in Inhalatoren, beeinträchtigt wird. Insbesondere kann es sich als schwierig erweisen, zur Freisetzung des Pulvers eine ausreichende bzw. stabile Öffnung in der Kapsel zu erzeugen. Ein gewisser Teil des Pulvers kann an der Kapselwandung haften bleiben und steht somit nicht für den therapeutischen Effekt nach Inhalation zur Verfügung. Zusammenfassend sind die mechanischen und physicochemischen Eigenschaften der im Stand der Technik bekannten Produkte, einschließlich von Produkten unter Verwendung der vorstehenden Kunststoffmaterialien nicht völlig zufriedenstellend.

WO 0007572 offenbart Kapseln, die sich an die Verwendung im Pulverinhalatoren anpassen und eine Kapselwandung aus einem nicht-wasserlöslichen Kunststoff aufweisen.

### Beschreibung der Erfindung

Eine der Hauptaufgaben der Kapsel ist es, den Wirkstoff wie auch die gesamte Inhalationsformulierung vor biologischer, chemischer oder physikalischer Veränderung zu schützen. Zu den physikalischen Veränderungen zählen dabei insbesondere Veränderungen, die das Ausbringen der vorbestimmten Feinpartikeldosis verändern können. Unter dem Begriff Feinpartikeldosis versteht man dabei die Dosis, die die Lunge des Patienten erreichen kann. Letztere wird von den Wechselwirkungen der mikronisierten Wirkstoffpartikel untereinander als auch der Wechselwirkungen mit den Hilfsstoffen beeinflusst. Es hat sich nun gezeigt, dass besonders durch Änderung des Feuchtigkeitsgrades im Inneren der Verpackung diese Wechselwirkungen derart zunehmen können, dass die Feinpartikeldosis deutlich vermindert ist. Derartige Veränderungen schließen dabei das Eindringen von Wasser in die Verpackung genauso ein, wie das Entfernen von Wasser aus dem Inneren der Verpackung.

Daher ist es eine Hauptaufgabe der Verpackung, die chemische Zusammensetzung der Atmosphäre im Inneren der Verpackung konstant zu halten, um physikalischen oder chemischen Veränderungen der Wirkstoffformulierung vorzubeugen, bzw. die Inhalationsformulierung stabil zu halten. In diesem Zusammenhang unterscheidet man einerseits zwischen einer auf eine kurze Zeit ausgerichteten Stabilität, die die Inhalationsformulierung während der Zeit der tatsächlichen Verwendung besitzen muss ("*in-use-Stabilität'),* d.h. die Zeit vom Herausnehmen der Kapsel aus der vorgesehenen zusätzlichen Schutzverpackung (Sekundärverpackung), Einsetzen in den Inhalator, bis hin zum Öffnen der Kapsel und Einatmen des Inhalationspulvers und andererseits der Langzeitstabilität, d.h. der Stabilität, die gewährleistet sein muss, solange die Inhalationsformulierung von der ungeöffneten Schutzverpackung (der Sekundärverpackung) sicher geschützt wird. Aufgabe der vorliegenden Erfindung war es weiterhin, eine Kapsel bereitzustellen, die bei verbesserter Schutzwirkung für die Wirkstoffformulierung einfach herzustellen ist, vorteilhafte mechanische und physico-chemische Eigenschften aufweist und die Nachteile des Standes der Technik vermeidet.

Eine Lösung dieses Problems konnte dadurch gefunden werden, dass Kapseln, wie sie z.B. im HandiHaler^{®} verwendet werden, aus einem Material hergestellt wurden, die eine Polymerzusammensetzung umfasst, in die mindestens ein Adsorbens eingearbeitet ist.

Überraschend hat sich dabei gezeigt, dass sich die Aufbewahrung von pulverförmigen, Inhalations-Formulierungen positiv auf die Feinpartikelverteilung auswirken können und sich sogar der Feinpartikelanteil der ausgebrachten Dosen erhöhen kann. Dabei wurde gefunden, dass dies sowohl die Langzeitstabilität der Arzneimittelformulierung erheblich verlängern kann, als auch die *in-use-Stabilität.*

Die vorliegende Erfindung betrifft daher Kapseln, die zusammen mit einem Pulverinhalator verwendet werden können und als solches integraler Bestandteil des einsatzfähigen Pulverinhalators sind. Dabei können die Kapseln sowohl in den Geräten bevorratet sein oder die Kapseln werden bei Verwendung einzeln manuell in das Gerät eingesetzt.

Eine Aufgabe der Kapsel und ihres Materials besteht bevorzugt darin, den Austausch von gasförmiger Materie, insbesondere Wasserdampf, zwischen ihrem Inneren und der Umgebung zu verzögern und ggf. zu minimieren.

Eine weitere Aufgabe besteht darin, ein Kapselmaterial zu finden, das geeignet ist, Kapseln vorzugsweise der Größe 3 herzustellen, die in einem Bernoulli-Inhalator eingesetzt werden können.

Eine weitere Aufgabe besteht darin, Kapseln mit einer für die Ausbringung aus den Inhalator angemessenen Gesamtmasse und mechanischen Stabilität herzustellen. Hierbei ist eine Dichte von Gelatine oder Polyethylen vorteilhaft.

### Beschreibung der Erfindung im Detail

Die Erfindung betrifft eine Kapsel, insbesondere zur Verpackung von Inhalationsformulierungen, bei der wenigstens ein Hohlraum von einer Wandung umschlossen ist, dadurch charakterisiert, dass zumindest ein Teil der Wandung eine Polymerzusammensetzung aufweist, die mindestens ein Adsorbens enthält.

Die Gestalt des erfindungsgemäßen Behältnisses wird durch die vorgesehene Verwendung, z.B. den zu verwendenden Pulverinhalator vorgegeben. Bevorzugt handelt es sich bei dem erfindungsgemäßen Behältnis um eine zweiteilige Kapsel, wobei die beiden Teile teleskopartig so ineinander hinein geschoben werden können, dass eine Kapsel mit einem darin befindlichen inneren, geschlossenen Hohlraum gebildet wird. In diesem Hohlraum befindet sich die Inhalationsformulierung. Bevorzugt ist die erfindungsgemäße Kapsel von zylinderartiger Gestalt mit abgerundeten Enden. Erst nach Öffnen der Kapsel im Inhalator, kann die pharmazeutische Zubereitung durch den Inhalationsvorgang ausgebracht werden. Die Kapsel ist bevorzugt integraler Bestandteil eines einsatzfähigen Inhalators.

In diesem Zusammenhang steht der Begriff "geschlossener Hohlraum" für einen durch Wandungen abgeschlossenen Hohlraum, aus dem ein darin befindliches Pulver nicht ohne Öffnen desselben entweichen kann.

Der Begriff "Inhalationsformulierung" steht hierbei bevorzugt für eine pharmazeutische Pulverformulierung, welches aus leicht zerstäubbaren Wirkstoff-Pulverpartikeln einer Größe (mittlerer aerodynamischer Durchmesser) von weniger als 100 Mikrometern, bevorzugt von 1 bis 15 Mikrometern, weiter bevorzugt von 1 bis 5 Mikrometern besteht. Zusätzlich kann die pharmazeutische Formulierung einen leicht fließenden, pulverförmigen Trägerstoff, z.B. Laktose, einer mittleren Größe <500µm, bevorzugt <200µm, besonders bevorzugt <100µm enthalten, sowie weitere Hilfssstoffe zur Verbesserung der Zerstäubbarkeit.

"Integraler Bestandteil eines einsatzfähigen Inhalators" bedeutet, dass die Kapsel bzw. ein Element in dem Inhalator vorhanden ist, ohne das die Beschickung des Inhalators mit der Arzneimittelformulierung (Inhalationsformulierung) zum Zweck der Inhalation nicht möglich ist bzw. nicht vorgesehen ist. Die Kapsel kann in gebrauchsfertigem Zustand (einsatzfähigen Zustand) fest mit dem Inhalator verbunden sein, so dass sie nicht zerstörungsfrei oder ohne den Inhalator zu beschädigen entnommen werden kann, oder sie ist zerstörungsfrei, lose bzw. lösbar mit dem Inhalator verbunden.

Einsatzfähig bedeutet, dass das erfindungsgemäße Behältnis in den Inhalator eingesetzt vorliegt. Dies kann bereits fabrikseitig erfolgen oder die Kapsel wird durch den Patienten in den Inhalator eingesetzt. Gegebenenfalls wird das Behältnis mechanisch durch Bauteile des Inhalators geöffnet und/oder in dem Inhalator zum Ort der Ausbringung transportiert. Die Reihenfolge dieser beiden Schritte kann auch verändert werden.

Mit den erfindungsgemäßen Kapseln wird eine Inhalationsformulierung gegenüber dem Eindringen von unerwünschten Substanzen, insbesondere von Feuchtigkeit von der Außenumgebung besser abgeschirmt als es bei den aus dem Stand der Technik bekannten, vergleichbaren Kapseln der Fall ist.

Die erfindungsgemäßen Kapseln enthalten eine Adsorbens-Polymerzusammensetzung, d.h. mindestens ein Teil der Kapsel ist aus einem solchen Material hergestellt. Nach einer bevorzugten erfindungsgemäßen Ausführungsform bestehen die Kapseln vollständig oder im Westentlichen aus einer Adsorbens-Polymerzusammensetzung, d.h. einer Polymerzusammensetzung enthaltend mindestens ein Adsorbens.

In der vorliegenden Beschreibung wird der Ausdruck Adsorbens verwendet, um eine Komponente zu bezeichnen, die in der Lage ist, mit einer chemischen, in der Kapsel (d.h. in der Nähe der pharmazeutischen Zusammensetzung) unerwünschten Komponente zu reagieren bzw. in Wechselwirkung zu treten, die eine Affinität für das Adsorbens aufweist, und die mit großer Wahrscheinlichkeit von dem Adsorbens zurückgehalten werden kann. Obwohl unterschiedliche Reaktionsmechanismen zu Grunde liegen, werden die Ausdrücke "Adsorbens" und "Absorbens" in der vorliegenden Beschreibung gleichbedeutend und austauschbar verwendet.

Als Adsorbens kann vorzugsweise jedes Material verwendet werden, das die Fähigkeit aufweist, Feuchtigkeit zu adsorbieren oder auf andere Weise Feuchtigkeit von der umgebenden Atmosphäre zu extrahieren, oder jedes Material, das in der Lage ist, aus der umgebenden Atmosphäre andere (unerwünschte) chemische Komponenten zu adsorbieren oder auf andere Weise zu extrahieren, worunter, ohne Beschränkung, auch Sauerstoff, Kohlendioxid, Kohlenmonoxid, Amine, Aldehyde, Epoxide und Alkohole fallen.

In der vorliegenden Beschreibung und den nachfolgenden Ansprüchen kann der Ausdruck "Adsorbens" nach einer bevorzugten Ausführungsform auch gleichbedeutend mit dem Ausdruck "Trockenmittel" oder "Entwässerungsmittel" verwendet werden. Nicht-beschränkende Beispiele geeigneter Adsorbentien umfassen u. a. Silicagele, trocknende bzw. Feuchtigkeit oder Wasser adsorbierende Tone, Alumosilicate wie Zeolithe oder Bentonite, Molekularsiebe, Aktivkohle, Erdalkalioxide, Calciumsulfat oder deren Mischungen.

Nach einer bevorzugten erfindungsgemäßen Ausführungsform umfasst das Adsorbens mindestens ein Entwässerungsmittel. Nicht-beschränkende Beispiele für bevorzugte Entwässerungsmittel sind Silicagel, Alumosilicate wie Bentonite oder Zeolithe, Molekularsiebe und/oder Calciumsulfat.

Die erfindungsgemäßen Kapseln verbessern die Brauchbarkeit der gefüllten Kapseln im Hinblick auf ihre Funktionalität, ihre Haltbarkeit bzw. Lagerstabilität, einschließlich der darin enthaltenen pharmazeutischen Zusammensetzungen. Insbesondere verhindern sie negative Auswirkungen von Feuchtigkeit oder anderer schädlicher chemischer Komponenten auf die pharmazeutischen Zusammensetzungen und das Kapselmaterial sowie eine Klebrigkeit der Kapselwände aufgrund erhöhter Feuchtigkeit.

Weiterhin wurde überraschend gefunden, dass die Anwesenheit mindestens eines Adsorbens bzw. Entwässerungsmittels in der Polymerzusammensetzung in vorteilhafter Weise ein Durchlöchern, Aufschneiden bzw. Öffnen der Kapselwandung auf andere Weise, insbesondere über Dorne oder Schneide-/Öffnungsvorrichtungen ermöglicht, wie sie z.B. in herkömmlichen Pulverinhalatoren verwendet werden, wobei ein Zerbrechen oder eine Fragmentierung der Kapseln vermieden wird. Somit hat das Adsorbens auch einen Einfluss auf die mechanischen Eigenschaften der Polymerzusammensetzung im Vergleich zu einer Polymerzusammensetzung ohne Adsorbens. Dies ermöglicht überraschend die Bereitstellung von Kapseln, die sowohl verbesserte Barriereeigenschaften aufweisen, als auch gleichzeitig die erforderliche Festigkeit und Durchdringbarkeit aufweisen, um z.B. mit Hilfe der Dorne oder Schneide-/Öffnungsvorrichtungen eine Öffnung in der Kapselwandung erzeugen zu können, die ausreichend groß und stabil ist, um die pulverförmige pharmazeutische Zusammensetzung wirkungsvoll freizusetzen. Es wurde unerwarteterweise auch gefunden, dass bei den erfindungsgemäßen Kapseln das Problem eines teilweisen oder vollständigen Wiederverschließens der Öffnung nach deren Erzeugung durch Aufschneiden oder Durchlöchern der Kapselwandung mit den Dornen oder Schneide-/Öffnungsvorrichtungen, aber vor der Freisetzung des Pulvers aus der Kapsel, weniger ausgeprägt ist oder sogar vollständig vermieden werden kann. Es wird angenommen, ohne dass die Erfindung auf die Richtigkeit dieser Annahme beschränkt wäre, dass dieser positive Effekt auf den Einfluss des Adsorbens auf die mechanischen und physikalischen Eigenschaften der Polymerszusammensetzung zurückzuführen ist. Somit verbinden die erfindungsgemäßen Kapseln einen stabilen und optimalen Schutz der Pulverzusammensetzung mit einer verbesserten Freisetzbarkeit der pharmazeutischen Zusammensetzung, auch bei anderen üblichen Verfahren zur Öffnung der Kapsel, z.B. unter Trennung der beiden Teile der Kapsel.

Vorzugsweise ist das in der Polymerzusammensetzung vorhandene Adsorbens ein teilchenförmiges Adsorbens. Das Adsorbens kann somit vorzugsweise in granulärer und Pulverform vorliegen. Jedoch sind auch polymere Adsorbentien im Rahmen der Erfindung brauchbar.

Nach einer besonders bevorzugten erfindungsgemäßen Ausführungsform enthält die polymere Zusammensetzung, bezogen auf Gew-% der Gesamtzusammensetzung, mindestens 5 Gew.-%, vorzugsweise mindestens 10 Gew.-%, insbesondere zwischen etwa 10 und 50 Gew.-% Adsorbens. Generell werden vorzugsweise bei höheren Wandstärken von mehr als etwa 0,4 mm etwas höhere Gehalte an Adsorbens verwendet, bspw. im Bereich von 10 bis 80 Gew.-%. Bei dünneren Kapselwandungen, vorzugsweise von weniger als etwa 0,4 mm, enthält die polymere Zusammensetzung vorzugsweise weniger als 50 Gew.-% Adsorbens. Selbstverständlich ist es möglich, die Dauer und Intensität des Schutzes der in der Kapsel verpackten pharmazeutischen Zusammensetzung vor schädlichen chemischen Komponenten wie Feuchtigkeit einzustellen, indem z.B. die Menge an Adsorbens in der Polymerzusammensetzung variiert wird. Auch kann über die Art des Adsorbens die Adsorptionskapazität und -kinetik eingestellt werden. Weiterhin kann z.B. über die Beschaffenheit der Polymerzusammensetzung, zusätzliche Additive, den Gehalt an Polymer, die Dicke der Kapselwandung und das zur Herstellung der Kapselwandungen verwendete Verfahren dessen Barrierefunktion eingestellt werden. Auch die spezifischen Austauschoberflächen zwischen der externen und der internen Atmosphäre der Kapsel können angepasst werden.

Nach einer bevorzugten erfindungsgemäßen Ausführungsform enthält die verwendete Polymerzusammensetzung (Polymerstruktur) mindestens ein thermoplastisches Material. Brauchbare thermoplastische Materialien sind allgemein alle Materialien mit thermoplastischen Eigenschaften, bspw. ein Polymer, das aus einem einzigen Monomer erhalten wurde, ein Copolymer aus zwei oder mehr Monomeren, ein Gemisch aus zwei oder mehr Polymeren aus je einem einzigen Monomer, ein Gemisch aus zwei oder mehr Copolymeren, oder ein Gemisch aus mindestens einem Polymer, das aus einem Monomer und mindestens einem Copolymer erhalten wurde.

Nach einer besonders bevorzugten erfindungsgemäßen Ausführungsform ist das verwendete thermoplastische Material Polyethylen oder Polypropylen. Nach einer weiteren erfindungsgemäßen Ausführungsform kann Polystyrol verwendet werden, insbesondere wenn ein Verschweißen (z.B. Ultraschallverschweißen) der Kapselteile gewünscht ist. Polyamide können bevorzugt sein wenn beispielsweise besonders dünne Kapselwandungen gewünscht sind. Um etwas "weichere" Eigenschaften einzustellen können z.B. auch Silicone verwendet werden.

Nicht-beschränkende Beispiele von Polymeren (thermoplastische Materialien) aus einzelnen Monomeren umfassen: Polystyrol, Polyolefine, insbesondere Polyethylen und Polypropylen, Polyacrylate, Polymethacrylate, Polyimide, Polycarbonate, Polyethersulfone, Polyamide, Polyester und Polyvinylchloride. Nicht-beschränkende Beispiele von Copolymeren umfassen: Ethylen-Acrylat, modifizierte Polymere und Maleinsäureanhydrid-Copolymere sowie gepfropfte Copolymere. Falls ein Copolymer oder ein Gemisch verwendet wird, wird die Verwendung einer Kombination bevorzugt, die aus Monomeren oder Polymeren besteht, die (mindestens) eine gemeinsame chemische Monomereinheit aufweisen. Beispielsweise kann ein thermoplastisches Material verwendet werden, das lineares Polyethylen geringer Dichte (LLDPE), Polyethylen mit geringer Dichte (LDPE) und Ethylen-Vinylacetat -Copolymer (EVA) enthält, worin jede der Komponenten Ethylen als Monomereinheit aufweist.

Nach einer möglichen erfindungsgemäßen Ausführungsform enthält die Polymerzusammensetzung kein Elastomer oder nur geringe Mengen davon. Es wurde gefunden, dass dadurch in vielen Fällen das Phänomen des Wiederverschließens der in der Kapselwand zur Freisetzung der pharmazeutischen Zusammensetzung erzeugten Öffnung besonders deutlich verringert werden kann. Nach einer alternativen Ausführungsform enthält die Polymerzusammensetzung jedoch mindestens ein Elastomer, wobei auch hier die Eigenschaften der Adsorbens-haltigen Polymerzusammensetzung besonders vorteilhaft für die Primärverpackung und Freisetzung von pulverförmigen pharmazeutischen Zusammensetzungen sind. Das Elastomer kann, bspw., aus der Gruppe bestehend aus Styrol-Butadien- Kautschuk (SBR), Styrol-Ethylen-Butadien-Styrol-Copolymeren (SEBS), Butyl-Kautschuk, Ethylen-Propylen-Kautschuk (EPR), Ethylen-Propylen-Dien-Monomer-Kautschuk (EPDM), und Ethylen-Vinylacetat-Copolymere (EVA) und Butadien-Acrylonitrilen ausgewählt werden.

Bevorzugte Materialien sind spritzguss- oder blasformtechnisch verarbeitbare Kunststoffe. Vorteilhaft sind zudem Kunststoffe, für deren Verarbeitung kein Formtrennmittel notwendig ist, das ein Anhaften des Füllguts an der Wand bewirken kann. Das hat den Vorteil, dass das Innere des Behältnisses nicht vom Formtrennmittel gereinigt werden muss, um z.B. den amtlichen Bestimmungen Genüge zu tun, die die Verwendung von Formtrennmitteln für Primärpackmittel einschränken.

Nach einer bevorzugten erfindungsgemäßen Ausführungsform kann die Polymerzusammensetzung enthalten oder bestehen aus 5 bis 85 Gew.-% mindestens eines Polymers, insbesondere mindestens eines thermoplastischen Materials, und 5 bis 85 Gew.% mindestens eines Adsorbens.

Die relative Konzentration von thermoplastischem Material und Adsorbens kann als Funktion des verwendeten thermoplastischen Materials und der verwendeten Adsorbentien variieren, vorzugsweise innerhalb der vorstehend angegebenen Bereiche. Nach einer besonders bevorzugten Ausführungsform enthält die Adsorbens-Polymerzusammensetzung Polyethylen, insbesondere hochdichtes Polyethylen (HDPE) und etwa 20 bis 50 Gew.%, insbesondere 20 bis 40 Gew.-% mindestens eines teilchenförmigen Adsorbens oder Entwässerungsmittels.

Das Adsorbens hat vorzugsweise eine maximale Teilchengröße von weniger als 50 µm, insbesondere weniger als 40 µm, besonders bevorzugt weniger als 25 µm, jeweils gemessen als max. 2% Siebrückstand. Die mittlere Teilchengröße, bestimmt nach der Laserbeugungsmethode (Malvern, nach Angaben des Herstellers, Messung in Luft) liegt zwischen etwa 0,5 und 50 µm, bevorzugt zwischen 0,5 und 40 µm, insbesondere zwischen 1 und 20 µm, weiter bevorzugt zwischen 1 und 10 µm.

Nach einer bevorzugten erfindungsgemäßen Ausfiihrungsform liegt die Wandstärke der Kapselwandung, zumindest in einem Abschnitt davon, zwischen etwa 0,05 und 2 mm, insbesondere zwischen etwa 0,1 und 1,1 mm, weiter bevorzugt zwischen 0,1 und 0,5 mm. Es wurde gefunden, dass eine solche Wandstärke in der Regel sowohl eine ausreichende Stabilität der Wirkstoffformulierung in der erfindungsgemäßen Kapsel bereitstellt, als auch eine vorteilhafte Freisetzbarkeit ermöglicht.

Weiterhin wurde gefunden, dass nach einer bevorzugten erfindungsgemäßen Ausführungsform das Verhältnis der maximalen Teilchengröße des Adsorbens zu der Wandstärke der Kapselwandung zwischen 0,01 und 0,2, insbesondere zwischen 0,02 und 0,1 liegen sollte, da hierbei besonders vorteilhafte Stabilitäten bei sehr guter Freisetzbarkeit der Wirkstoffformulierungen gefunden wurden.

Nach einer bevorzugten erfindungsgemäßen Ausführungsform wird die Grundmischung mit dem mindestens einem Polymer und dem mindestens einen Adsorbens auf herkömmliche Weise hergestellt, indem das Polymer (thermoplastische Material) erhitzt wird, um eine Schmelze zu erzeugen, das Adsorbens (soweit noch nicht geschehen) und andere herkömmliche Additive zugefügt und eingemischt werden, worauf das Gemisch in ein Granulat überführt und in dieser Form gelagert werden kann. Dieses Granulat kann dann zur Herstellung der gewünschten Struktur (Kapsel) verwendet werden, bspw. durch Extrusionsformen, insbesondere jedoch durch Spritzgießen.

Nach einer bevorzugten erfindungsgemäßen Ausführungsform ist das mindestens eine Adsorbens gleichmäßig über die Stärke der Kapselwandung oder des aus der Polymerzusammensetzung mit mindestens einem Adsorbens hergestellten Teils davon verteilt. Bei dieser Ausführungsform kann die polymere Zusammensetzung als monolithisch angesehen werden.

Nach einer anderen bevorzugten erfindungsgemäßen Ausführungsform ist das mindestens eine Adsorbens jedoch nahe der Oberfläche der Kapselwandung bzw. Polymerzusammensetzung in einer höheren Konzentration vorhanden als in deren inneren Bereich. Demgemäß weist die verwendete Polymerzusammensetzung mindestens eine Wanderungszone an einer Oberfläche des Kapselwandung und einen inneren Bereich auf, wobei die maximale Konzentration des mindestens einen Adsorbens innerhalb der Wanderungszone mindestens doppelt so hoch ist wie die maximale Konzentration des mindestens einen Absorbens in dem inneren Bereich. Dies ist in vielen Fällen vorteilhaft im Hinblick auf die Barrierefunktion der Kapselwandung. Solche polymeren Strukturen sind bspw. in der PCT/FR03/03465 oder PCT/IB2004/004403 beschrieben. Deren diesbezügliche Offenbarung wird hiermit ausdrücklich durch Bezugnahme in die Beschreibung aufgenommen.

Allgemein kann jedoch nach einer möglichen Ausführungsform der vorliegenden Erfindung auch jegliche andere Adsorbens-Polymerzusammensetzung im Rahmen der vorliegenden Erfindung verwendet werden, bspw. die in der US 5,432,214, der US 5,911,937, der US 4,665,050, der EP 0 432438 oder der EP 0 400 460 beschriebenen. Deren entsprechende Offenbarung wird hiermit ausdrücklich durch Bezugnahme in die vorliegende Beschreibung aufgenommen.

Wie bereits erwähnt, können die verwendeten Adsorbens-Polymermaterialien weiterhin (herkömmliche) organische oder anorganische Zusätze (Additive) wie Fasern, Stabilisatoren, Farbstoffe, Pigmente, Expansionsmittel, oder Kombinationen daraus enthalten oder auch andere aus der Kunststoffverarbeitung bekannte Zusätze. Bevorzugt wird die Menge an diesen zusätzlichen Stoffen auf eine Mindestmenge reduziert. Nach einer bevorzugten Ausführungsform enthält die Adsorbens-Polymerzusammensetzung mindestens eine adsorbierende Faserkomponente, insbesondere eine Superabsorber-Faser, z.B. aus Polyacrylat. Nach einer anderen erfindungsgemäßen Ausführungsform werden in der Polymerzusammensetzung keine aufsaugenden Fasern (wicking fibers) verwendet.

In einer bevorzugten Ausfükungsform besitzt der entwässernde Kunststoff keine ausgeprägte Adhäsion für pharmazeutisch-chemische Stoffe, insbesondere für Partikel mit lungengängiger Größe, so dass bei Verwendung des Behältnisses in einem Inhalator der gesamte Inhalt der Kapsel freigesetzt werden kann. Dies gewährleistet eine exaktere Dosierung, insbesondere des lungengängigen Feinanteils der pharmazeutischen Zubereitung.

Weitere Angaben zu möglichen polymeren Zusammensetzungen oder die mögliche Verarbeitung betreffend können dem vorstehend genannten Stand der Technik entnommen werden, insbesondere der EP-A 0 599 690 und der PCT/IB2004/004403.

In einer Ausführungsform kann die Behälterwandung Regionen mit unterschiedlicher Zusammensetzung aus Polymer / Adsorbens enthalten. Letzteres bietet sich z.B. bei Kapseln an, die aus mehreren unterschiedlichen Teilen bestehen.

In einigen Ausführungsformen besteht die Wandung der Kapsel aus wenigstens zwei Schichten, einer inneren und wenigstens einer darüber liegenden äußeren Schicht. Dabei bildet die innere Schicht die unmittelbare Wandung des Hohlraums und steht damit in Kontakt mit der Inhalationsformulierung. In diesem Fall kann eine der beiden Schichten aus der Polymerzusammensetzung mit Adsorbens bestehen, die andere Schicht aus einem pharmakologisch neutralen Material. Möglich sind auch Sandwichstrukturen, bei denen die äußerste Schicht eine erste Barriere gegen Feuchtigkeit bildet, die mittlere Schicht aus dem Polymer mit Adsorbens und die innere Schicht aus einem pharmakologisch neutralen Material besteht. Andere Schichtabfolgen sind möglich. Geeignete pharmakologisch neutrale Materialien sind dem Fachmann geläufig und umfassen (neben den erfindungsgemäß verwendeten Polymerzusammensetzungen mit mindestens einem Adsorbens) beispielsweise Polymerzusammensetzungen oder Kunststoffe wie Polyethylen, Polypropylen oder Polystyrol.

Dabei können die Materialien der einzelnen Schichten so gewählt werden, dass sie nicht stoffschlüssig miteinander verbunden werden oder dass sie stoffschlüssig miteinander verbunden werden.

In wieder einer anderen Ausführungsforrn, wird die Wandung des Behältnisses von Außen mit der das Adsorbens enthaltenden Polymerzusammensetzung beaufschlagt, so dass eine geschlossene Siegelfläche entsteht. Diese Variante hat den Vorteil, dass mit Aufbringen der absorbierenden, insbesondere der entwässernden Schicht auch eventuelle Nahtstellen, die die Wandungen der Kavitäten aufweisen können, versiegelt werden. Diese Variante eignet sich besonders bei zweiteiligen Kapseln.

Die erfindungsgemäß bevorzugte Kapsel ist eine zweiteilige Kapsel.
Derartige Kapseln bestehen bevorzugt aus zwei, teleskopartig ineinander einschiebbaren Teilen, einem Kapselkörper (Körper) und einer Kapselkappe (Kappe), die so miteinander verbunden werden können, dass ein stabiler, abgeschlossener Hohlraum von definiertem Volumen gebildet wird, der die pharmazeutische Zubereitung beinhaltet. Die Dimension der Kapsel ist derart, dass sie z.B. in gängigen mit Kapseln bestückten Pulverinhalatoren eingesetzt werden kann.

In einer besonders bevorzugten Ausführungsform haben die Kappe und der Körper der Kapsel die Form eines Zylinders mit rundem Querschnitt und konvex, nahezu halbkugelförmigen geschlossenen Stirnflächen.

In einer bevorzugten Ausführungsform besteht die Kapsel aus einem entwässernden Kunststoff mit einer Shorehärte D von 60 bis 80, bevorzugt von 65 bis 73.

Es wird bevorzugt, dass eine solche Kapsel so stabil ist, dass sie entlang der Längsachse oder der Querachse einer Kraft bis zu 10 N, weiter bevorzugt von bis zu 15 N standhält. Der Vorteil besteht darin, dass die Kapsel besser an die Beanspruchungen angepasst ist, die bei der Herstellung, dem Füllen, Verpacken, Transportieren u.ä. auf die Kapsel einwirken.

In einer bevorzugten Ausführungsform sind Kappe und Körper der Kapsel von gegenseitig ähnlicher, zylinderartiger Form, bestehend aus einem in sich geschlossenen Mantel mit jeweils einer geschlossenen und einer offenen Seite. Dabei sind Form und Größe der Kappe und der Kapsel dergestalt, dass der Körper mit seinem offenen Ende teleskopartig so in das offene Ende der Kappe hinein geschoben werden kann, dass die Kappe fest mit dem Körper verbunden ist.

In einer speziellen Ausführungsform sind Kappe und Körper mit Verschlusseinrichtungen versehen, die beim vorläufigen und/oder endgültigen Verschließen der Kapsel von Vorteil sind.
In einer solchen Ausführungsform befinden sich auf dem Innenmantel der Kappe punktförmige Erhebungen und auf dem Außenmantel des Körper etwas größere punktförmige Vertiefungen, die so angeordnet sind, dass beim Verschließen der Kapsel die Erhebungen in die Vertiefungen einrasten. Alternativ können die Erhebungen auf dem Außenmantel des Körpers und die Vertiefungen auf dem Innenmantel der Kappe ausgebildet sein. Bevorzugt sind Anordnungen, bei denen die Erhebungen oder Vertiefungen jeweils ringförmig oder spiralförmig um den Mantel angeordnet sind. Anstelle der punktförmigen Gestaltung der Erhebungen und Vertiefungen können diese auch durchgehend den Mantel der Kappe bzw. des Körpers ringförmig umlaufen.

In einer Ausführungsform sind auf dem Innenmantel der Kappe und dem Außenmantel des Körpers ein oder mehrere ringförmig umlaufende Erhebungen derart ausgebildet, dass sich im geschlossenen Zustand der Kapsel eine Erhebung der Kappe jeweils neben einer Erhebung des Körpers befindet.

In den Ausführungsformen mit besagten ringförmigen Vertiefungen und/oder Erhebungen können diese durchgängig oder unterbrochen sein.
In einer weiteren Ausführungsform sind auf der Außenseite des Körpers nahe dem offenen Ende Erhebungen und in der Kappe nahe dem offenen Ende Löcher so ausgebildet, dass die Erhebungen des Körpers im geschlossenen Zustand der Kapsel in die Löcher der Kappe einrasten. Die Erhebungen können dabei derart sein, dass die Kappe jederzeit ohne Beschädigung der Kapsel geöffnet werden kann oder aber dass die Kapsel nach einmaligem Verschließen nicht mehr zerstörungsfrei geöffnet werden kann.

In einer weiteren Ausführungsform ist auf der Außenseite des Körpers ein Wulst ausgebildet, der senkrecht zu der Verbindungsachse zwischen Kappe und Körper rings um den Körper läuft. Der Wulst dient als Stopper für die Kappe, wenn diese über den Körper gesteckt wird, um ein Durchstoßen der Kappe mit dem Körper zu verhindern. Der Bereich zwischen offenem Ende des Körpers und dem Wulst entspricht dem Bereich des Körpers über den die Kappe geschoben werden kann. Der Wulst ist so auf dem Körper lokalisiert, dass die Kappe weit genug über den Körper geschoben werden kann, um einen festen Verschluss zwischen Kappe und Körper zu bewirken. D.h. der Wulst befindet sich z.B. nicht unmittelbar an der offenen Seite des Körpers. Die Seite des Wulstes, die zum offenen Ende des Körpers zeigt, steht als senkrechte Kante so auf der Außenwand des Körpers, dass die Kappe beim Verschließen nicht über den Wulst hinweg geschoben werden kann. Die Seite des Wulstes, die zum geschlossenen Ende des Körpers weist, kann in Form einer nahezu rechtwinkeligen Kante ausgebildet sein oder sich zum geschlossenen Ende des Körpers hin verflachen. Die Ausbildung einer nahezu rechtwinkeligen Kante kann bei einer losen Einpassung der Kapsel in einen Kapselhalter von Vorteil sein, die Variante mit sich verflachendem Wulst bei einer festen Einpassung. Der Wulst kann durchgängig oder unterbrochen sein.

In einer bevorzugten Ausführungsform verflacht sich der Wulst kontinuierlich zum geschlossenen Ende des Körpers und steht mit seiner zum offenen Ende des Körpers orientierten Seite senkrecht auf dem Kapselkörper auf. Dabei ist die Höhe der so gebildeten Kante derart, dass die Kante im geschlossenen Zustand der Kapsel nicht über die Kapselkappe hinausragt, so dass der Übergang von Kapselkappe zu Kapselkörper plan ist.

Die Stärke der Wände der Kappe und des Körpers können über den Gesamtbereich variieren. So ist die Wandstärke in der Regel in den abgerundeten Bereichen der Kappe oder des Körpers oder an der Stelle des Körpers, an der der Wulst ausgebildet ist größer als in den Bereichen, in denen die Wände geradlinig verlaufen. In einer Ausführungsform haben die Wände der Kappe und des Körpers eine Dicke von 0,1 mm bis 0,5 mm.

In einer weiteren Ausführungsform sind an der Außenseite der Kapsel Noppen ausgebildet, in einer anderen drei oder mehr Rippen, die parallel zur Längsachse der Kapsel verlaufen. Der Vorteil dieser Einrichtungen besteht darin, dass die Kapsel aus einer Kapselhalterung, wie sie z.B. in den oben genannten Pulverinhalatoren verwendet werden, so herausgenommen werden kann, dass sie nicht beschädigt wird oder aufgeht. Die Rippen oder Noppen können über die gesamte Außenseite der Kapsel hinweg verlaufen oder nur einen Teil davon bedecken. Alternativ können sie nur an der Kappe ausgebildet sein oder nur in dem Bereich des Körpers, der im geschlossenen Zustand der Kapsel nach außen sichtbar ist. Die Rippen verlaufen parallel zur Längsachse der Kapsel und bewirken, dass die Kapsel senkrecht in besagter Kapselhalterung fixiert ist. Im Fall eines kreisförmigen Querschnitts der Kapsel sind die Rippen bevorzugt so angeordnet, dass der Querschnitt der Kapsel keine Rotationssymmetrie um die Mittelachse aufweist. In einer solchen Ausführungsform können die Rippen nur in dem Bereich des Körpers ausgebildet sein, der im geschlossenen Zustand der Kapsel sichtbar ist. Eine solche Ausführungsform verhindert das Festklemmen der Kapsel in einen Kapselhalter.

In einer Ausführungsform ohne Wulst, aber mit Rippen an dem Teil des Körpers, der im geschlossenen Zustand der Kappe sichtbar ist, sind die Rippen so ausgebildet, dass die zum offenen Ende des Körpers orientierten Enden der Rippen die Aufgabe des Wulstes erfüllen, nämlich als Stopper für die Kappe zu dienen beim Zusammenfügen der Kappe mit dem Körper.

In einer weiteren Ausführung beschreiben die Mäntel der Kappe und des Körpers einen hohlen Zylinder mit rundem, ovalem, drei-, vier-, sechs-, acht- oder mehreckigem Querschnitt, wobei die jeweilige Oberseite offen und die Unterseite geschlossen ist. Die geschlossene Unterseite kann flach oder konvex sein. Die eckigen Ausführungsformen haben z.B. den Vorteil, dass sie Platz sparend gelagert werden können.

In einer Ausführungsform ist die Elongation der Kapsel (Abstand vom geschlossenen Ende des Körpers zum geschlossenen Ende der Kappe in Relation zum Durchmesser bei geschlossener Kapsel) größer 1, in einer weiteren Ausführungsform ist die Elongation gleich 1 und in wieder einer anderen Ausführungsform ist die Elongation kleiner 1. Letzteres hat den Vorteil, dass der Körper eine größere Öffnung zum Füllen aufweist.
Bei einer der Ausführungsformen mit einer Elongation gleich 1 sind Kappe und Körper dergestalt, dass die geschlossene Kapsel die Form einer Kugel hat, was für ein automatisches Beladen eines Inhalators mit der Kapsel aus einem Reservoir von Vorteil sein kann.

Um im geschlossenen Zustand der gefüllten Kapsel eine bessere Abdichtung zwischen Kappe und Körper zu erreichen, kann die Nahtstelle zwischen Kappe und Körper zum Verschließen verschweißt, verklebt oder banderoliert werden, wodurch die Wasserdampfpermeabilität erheblich abnimmt. Bevorzugt ist das Verschweißen von Kappe und Körper. Alternativ kann die gesamte Kappe mit einem durchgehenden Schutzfilm überzogen werden. Letzterer kann aus dem erfindungsgemäß bevorzugten Material sein. In diesem Fall kann die Kapsel selbst aus einem Material sein, welches kein Adsorbens enthält.

In einer weiteren bevorzugten Ausführungsform kann der Spalt mit einem Füllstoff verschlossen werden. Als Füllstoff für ein solches Verfüllen des Spalts eignen sich pharmazeutisch zulässige Füllmaterialien, wie beispielsweise Eudragit, einer Polymerfamilie auf Basis von Methacrylsäure und Methacrylsäureestern. Ein solcher Füllstoff kann als Lösung oder Suspension in einem geeigneten, bevorzugt leicht flüchtigen, Lösungsmittel in den Spalt eingetragen werden.

Derartige Kapseln sind geeignete Behältnisse der erfindungsgemäßen Art für Inhalatoren wie sie beispielsweise unter den Markennamen HandiHaler^{®}, Spinhalere^{®}, Rotahaler^{®}, Aerolizer^{®}, Flowcaps^{®}, Turbospin^{®}, AIR DPI^{®}, Orbital bekannt sind und/oder in DE 33 45 722, EP 0 591 136, DE 43 18 455, WO 91/02558, FR-A-2 146 202, US-A-4 069 819, EP 666085, US3991761, WO99/45987 beschrieben werden.

Eine bevorzugte Erfindung betrifft ein Ensemble aus einem Inhalator für die Inhalation pulverförmiger Arzneimittel und der erfindungsgemäßen Kapsel, insbesondere eine zweiteilige Kapsel von zylinderartiger Gestalt mit abgerundeten Enden, wobei der Inhalator gekennzeichnet ist durch a) ein nach oben hin offenes, becherförmiges Unterteil, welches in der Ummantelung zwei gegenüberliegende Fenster aufweist und am Rand der Öffnung ein erstes Scharnierelement hat, b) eine Platte, welches die Öffnung des Unterteils bedeckt und ein zweites Scharnierelement aufweist, c) eine Inhalationskammer zum Aufnehmen der Kapsel, die senkrecht zur Plattenebene an der zum Unterteil weisenden Seite der Platte ausgebildet ist und an der ein gegen eine Feder beweglicher Kopf vorgesehen ist, wobei der Kopf mit zwei geschliffenen Nadeln versehen ist, d) ein Oberteil mit einem Mundrohr und einem dritten Scharnierelement, sowie e) einen Deckel, der ein viertes Scharnierelement aufweist, wobei die Scharnierelemente des Unterteils, der Platte, des Oberteils und des Deckels miteinander verbunden sind.
Bevorzugt handelt es sich hierbei um einen Inhalator der Marke HandiHaler^{®}.

Ein weiterer Aspekt der vorliegenden Erfindung betrifft ein Sekundärpackmittel, enthaltend mindestens eine Kapsel wie hierin beschrieben, wobei das Sekundärpackmittel vorzugsweise feuchtigkeitsdicht ist und weiter bevorzugt zumindest teilweise eine Aluminiumfolie aufweist (z.B. ein Blister).

Noch ein weiterer Aspekt der vorliegenden Erfindung betrifft das Trocknen von kleinen Mengen inhalationsfähiger Arzneimittelformulierungen. Es hat sich nämlich gezeigt, dass die erfindungsgemäßen Kapseln nicht nur in der Lage sind, den Eintritt von Feuchtigkeit in die Kapseln zu verhindern oder zu verzögern, so dass über die Kapseln ein Verfahren zur Trockenhaltung von inhalationsfähigen Arzneistoffformulierungen zugänglich wird, sondern auch, dass mit den erfindungsgemäßen Kapseln inhalationsfähiger Arzneimittelformulierungen mit geringer Restfeuchte getrocknet werden können. Besonders geeignet sind Arzneimittelformulierungen in einer Menge von bis zu 50 mg, bevorzugt bis zu 30 mg, besonders bevorzugt bis zu 15 und ganz besonders bevorzugt bis zu 10 mg. Der Grad an Feuchtigkeit kann z.B. bis zu 5 Gew. % der Formulierung ausmachen. Die Angaben beziehen sich auf Kapseln der Größe 3. Diese und weitere geeignete Kapselgrößen werden bei den Beispielen näher beschreiben.

Schließlich betrifft ein weiterer Aspekt der vorliegenden Erfindung die Verwendung einer Polymerzusammensetzung wie hierin beschrieben (mit mindestens einem Adsorbens) zur Herstellung einer Kapsel wie hierin beschrieben.

### Beispiele

Kapseln der Größe 3 wurden wie folgt hergestellt:
Die Adsorbens-Polymerzusammensetzung enthielt 67 Gew.-% High-Density Polyethylen (HDPE), 3 Gew.-% EVA (Elastomer) und 30 Gew.-% eines synthetischen Zeoliths (Molecular Sieve 4A, max. Teilchengröße 20 µm). Zunächst wurde manuell eine Vormischung aus HDPE und EVA hergestellt. Diese wurde in einem DoppelschneckenExtruder mit dem synthetischen Zeolith vermischt. Das Kompound wurde in Granulatform überführt. Das Granulat kann, soweit es nicht sofort weiter verarbeitet wird, zur Lagerung in Aluminiumbeutel verschweißt werden, um Feuchtigkeit auszuschließen. Das Granulat wurde mittels Spritzguß zu Kapseln der Größe 3 verarbeitet, wobei ein MFI (Melt Flow Index) von etwa 18 eingestellt und ein Injektionskanal mit 0,6 mm verwendet wurde. Nach einer zweiten Variante enthielt die Adsorbens-Polymerzusammensetzung 66 Gew.-% High-Density Polyethylen (HDPE), 3 Gew.-% EVA (Elastomer), 30 Gew.-% eines synthetischen Zeoliths (Molecular Sieve 4A, max. Teilchengröße 20 µm) und zusätzlich 1 Gew-% einer synthetischen Polyacrylat-Superabsorber-Faser.

Die erhaltenen Kapseln wurden mit 5,5 mg einer wasserempfindlichen Formulierung gefüllt und anschließend wurden Kapselober- und -unterteil miteinander verschweißt.
Die so hergestellten Kapseln wurden offen, also ohne weitere Sekundärverpackung bei einer Temperatur von bei 40°C und 75% relativer Luftfeuchte gelagert. Nach verschiedenen Zeiten wurde der inhalierbare Anteil der Partikel (Masseanteil der Partikel mit einer aerodynamischen Größe kleiner 5 µm) bei Ausbringung mit einem Pulverinhalator gemessen. Als Pulverinhalator kam ein Gerät des Typs HandiHaler^{®} (WO94/28958) zum Einsatz, der inhalierbare Anteil wurde mit Hilfe eines Kaskadenimpaktors bei einer Flußrate von 39 1/min gemessen.

Als Ergebnis wurde gefunden, dass die "in-use Stabilität" der Formulierung in Kapseln, die aus herkömmlichem Material ohne Zusatz von Molekularsieb bestehen, von 1 Tag um mindestens 9 Tage verlängert werden konnte. Als "in-use Stabilität" ist der Zeitraum zwischen der Herausnahme der Kapsel aus der Verpackung (z.B. der Blisterverpackung) und der Verwendung der Kapsel beim Inhalationsvorgang zu verstehen. In diesem Zeitraum muß die Kapsel eine ausreichende Stabilität aufweisen, um den in ihr enthaltenen Wirkstoff z.B. gegen Feuchte zu schützen. Dieses ist insbesondere bei Mehrdosisinhalatoren von großer Bedeutung.

Die erfindungsgemäßen Kapseln sind bevorzugt von folgender Größe:
a) Länge der Kapselkörper: 22,2 ±0,46 mm; 20,22 ±0,46 mm; 20,98 ±0,46 mm; 18,4 ±0,46 mm; 16,61 ±0,46 mm; 15,27 ±0,46 mm; 13,59 ±0,46 mm; 12,19 ±0,46 mm; 9,3 ±0,46 mm.
b) Länge der Kapselkappe: 12,95 ±0,46 mm; 11,74 ±0,46 mm; 11,99 ±0,46 mm; 10,72 ±0,46 mm; 9,78 ±0,46 mm; 8,94 µ±0,46 mm; 8,08 ±0,46 mm; 7,21 ±0,46 mm; 6,2 ±0,46 mm.
c) Äußerer Durchmesser der Kapselkörper: 9,55 mm; 8,18 mm; 7,36 mm; 7,34 mm; 6,63 mm; 6,07 mm; 5,57 mm; 5,05 mm; 4,68 mm.
d) Äußerer Durchmesser der Kapselkappen: 9,91 mm; 8,53 mm; 7,66 mm; 7,64 mm; 6,91 mm; 6,35 mm; 5,83 mm; 5,32 mm; 4,91 mm.
e) Gesamtlänge der geschlossenen Kapsel: 26,1 ±0,3 mm; 23,3 ±0,3 mm; 24,2 ±0,3 mm; 21,7 ±0,3 mm; 19,4 ±0,3 mm; 18,0 ±0,3 mm; 15,9 ±0,3 mm; 14,3 ±0,3 mm; 11,1 ±0,3 mm.
f) Kapselvolumina: 1,37 ml; 0,95 ml; 0,78 ml; 0,50 ml; 0,37 ml; 0,30 ml; 0,21 ml; 0,13 ml.

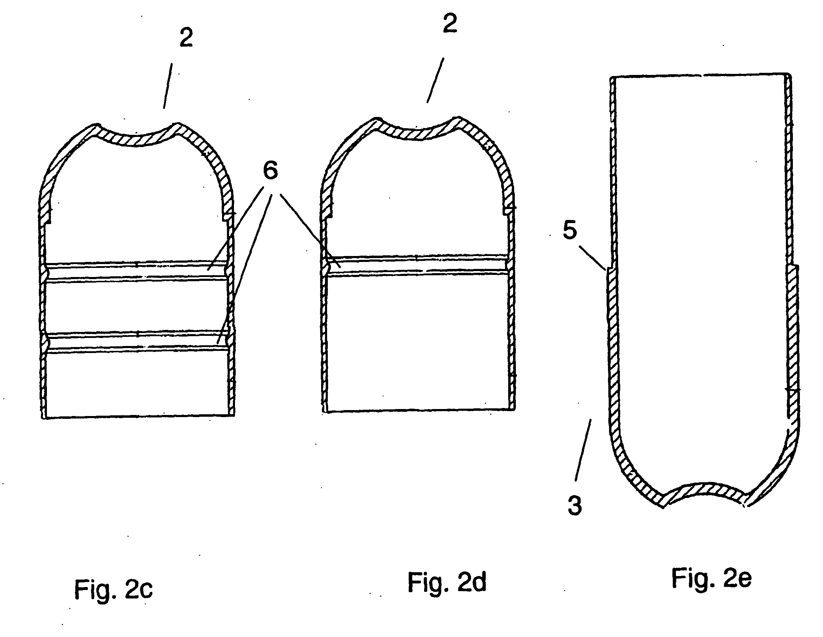

Nachfolgend soll die Erfindung anhand von Zeichnungen näher erläutert werden:

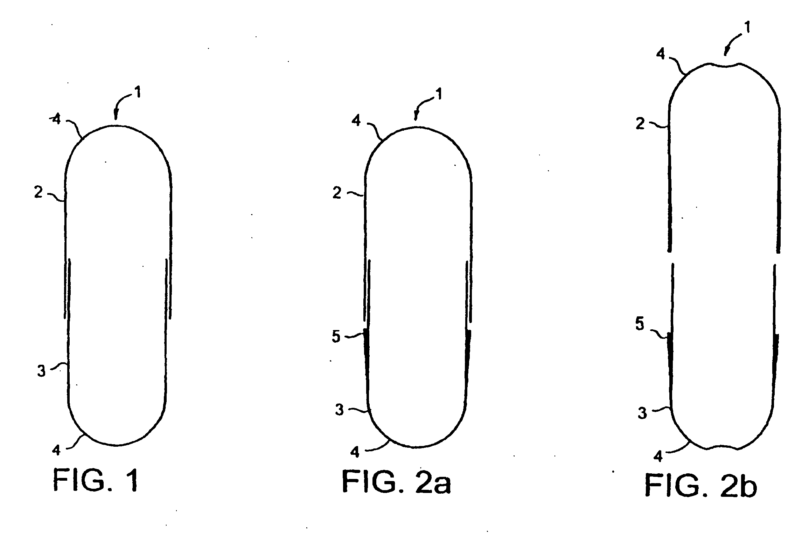

In Figur 1 ist die einfachste Ausführungsform der erfindungsgemäßen Kapsel **1** im Querschnitt gezeigt. Die Kapsel **1** besteht aus der Kappe **2** und dem Körper **3**, die teleskopartig ineinander gesteckt sind. Kappe **2** und Körper **3** sind von gleicher Gestalt und haben je eine konvexe Unterseite **4**.
   In Figur 2a ist im Querschnitt eine Ausführungsform gezeigt, bei der am Körper **3** der Kapsel **1** ein Wulst **5** ausgebildet ist, der sich zum geschlossenen Ende des Körpers hin verjüngt. Mit der zum offenen Ende des Körpers hin orientierten Seite steht der Wulst **5** nahezu senkrecht auf dem Körper. Die so ausgebildete Kante begrenzt den Bereich des Körpers über den die Kappe **2** teleskopartig geschoben werden kann.
   Eine andere Ausführungsform ist in Figur 2b abgebildet. Der Querschnitt zeigt, daß sich diese Ausführungsform von der in Figur 2a dargestellten dadurch unterscheidet, daß die Wandstärke der Kappe **2** bzw. des Körpers **3** nicht über den gesamten Bereich gleich stark ausgebildet ist, sondern über einzelne Teilbereiche variiert. Zusätzlich weisen die konvexen Unterseiten **4** der Kappe bzw. des Körpers je eine konkave Einbuchtung am Scheitelpunkt auf. In den Figuren 2c und 2d sind Kapselkappen mit Erhebungen auf dem Innenmantel **6** dargestellt. Figur 2e zeigt eine weitere Ausführungsform des Kapselkörpers, die sich von dem der Figuren 1, 2a und 2b durch die Dicke der Wandstärke unterscheidet.

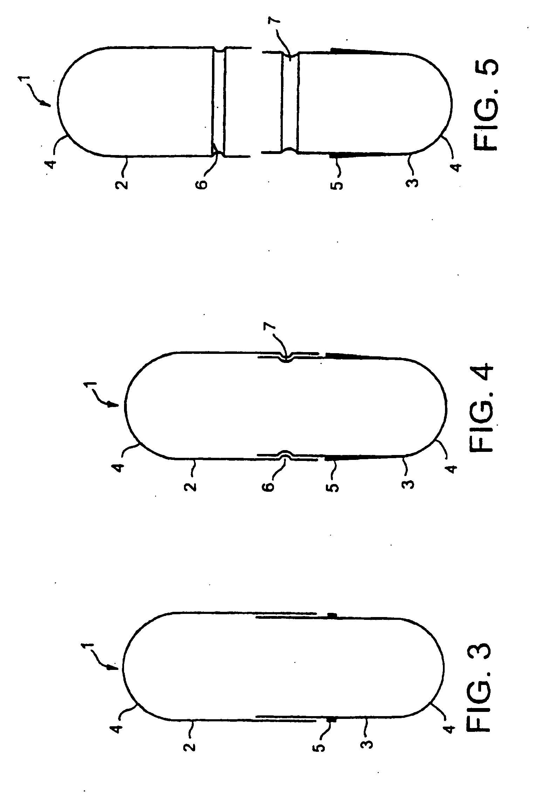

   In Figur 3 ist eine Ausführungsform dargestellt, bei der der Wulst **5** sowohl zur Oberseite des Körpers als auch zu seiner Unterseite nahezu rechtwinkelig auf dem Körper aufsitzt.
   Die Ausführungsform der Figur 4 stellt eine Weiterentwicklung der Ausführungsform der Figur 2a dar, bei der eine ringförmige Vertiefung bzw. Erhebung **6** bzw. **7** in Kappe **2** bzw. Körper **3** zum besseren Verschluss der Kapsel **1** ausgebildet ist.
   In Figur 5 ist eine Frontalansicht der in Figur 4 als Querschnitt gezeigten Ausführungsform abgebildet.

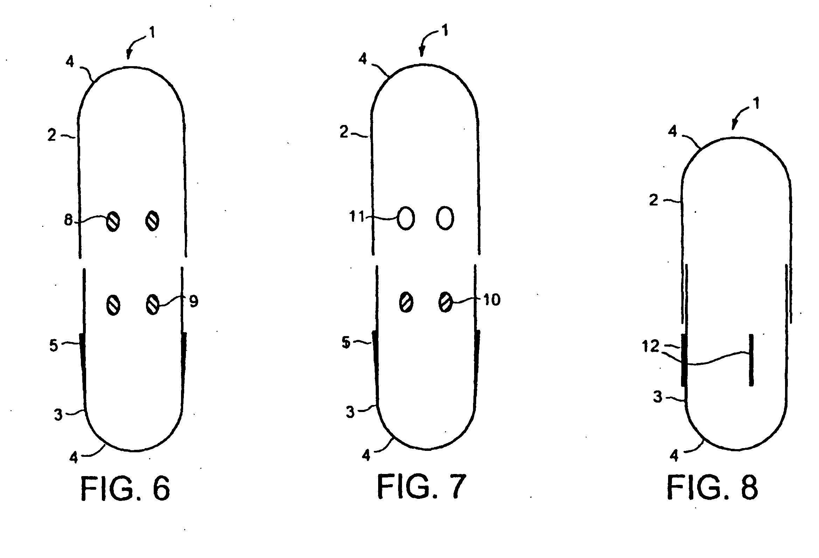

   Figur 6 zeigt eine weitere Variante der Erfindung mit punktuellen Vertiefungen **8** und **9** als Frontalansicht.
   In Figur 7 ist eine Variante der Kapsel **1** dargestellt, bei der am Körper **3** nahe dem offenen Ende Erhebungen **10** und in der Kappe 2 nahe dem offenen Ende Löcher **11** so ausgebildet sind, daß die Erhebungen **10** beim Verschließen der Kapsel in die Löcher **11** einrasten.
   Die Figur 8 stellt eine Ausführungsform der Kapsel **1** von außen dar, bei der auf dem Körper **3** die Rippen **12** ausgebildet sind.

Die Kapseln, wie sie in den Figuren dargestellt sind, können in einer Ausführungsform aus dem erfindungsgemäßen Material, besonders aus Polyethylen mit Entwässerungsmittel hergestellt werden. In einer anderen Ausführungsform wird die Kapsel aus einem Polymer hergestellt und anschließend mit diesem Material überspritzt. In einer anderen Ausführungsform wird die Kapsel anschließend mit einer Metallfolie z.B. Aluminiumfolie banderoliert oder laminiert.

## Patentansprüche

1. Kapsel, insbesondere zur Verpackung für Inhalationsformulierungen, bei der wenigstens ein Hohlraum von einer Wandung umschlossen ist, **dadurch** charakterisiert, dass zumindest ein Teil der Wandung eine Polymerzusammensetzung aufweist, die mindestens ein Adsorbens enthält.

2. Kapsel nach Anspruch 1, **dadurch gekennzeichnet, dass** sie durch wenigstens zwei teleskopartig ineinander einsteckbare zylinderartige Teilelemente gebildet wird, wobei die Kapsel integraler Bestandteil eines gebrauchsfertigen Pulverinhalators ist.

3. Kapsel nach Anspruch 1 oder 2, **dadurch gekennzeichnet, dass** zumindest ein Teil der Wandung überwiegend, im wesentlichen oder vollständig aus der Polymerzusammensetzung mit mindestens einem Adsorbens besteht.

4. Kapsel nach einem der vorangegangenen Ansprüche, **dadurch gekennzeichnet, dass** die Polymerzusammensetzung mindestens ein thermoplastisches Material enthält.

5. Kapsel nach einem der vorangegangenen Ansprüche, **dadurch gekennzeichnet, dass** das thermoplastische Material mindestens ein Polyolefin umfasst, besonders bevorzugt Polyethylen oder Polypropylen.

6. Kapsel nach einem der vorangegangenen Ansprüche, **dadurch gekennzeichnet, dass** das Absorptionsmittel in Teilchenform vorliegt, insbesondere mit einer maximalen Teilchengröße von weniger als 50 µm, insbesondere weniger als 40 µm, besonders bevorzugt weniger als 20 µm.

7. Kapsel nach einem der vorangegangenen Ansprüche, **dadurch gekennzeichnet, dass** die Wandstärke der Kapselwandung zumindest in einem Abschnitt davon zwischen etwa 0,05 und 2 mm, insbesondere zwischen etwa 0,1 und 1,1 mm, weiter bevorzugt zwischen 0,1 und 0,5 mm liegt.

8. Kapsel nach einem der vorangegangenen Ansprüche, **dadurch gekennzeichnet, dass** das Verhältnis der Teilchengröße des Absorptionsmittels zu der Wandstärke der Kapselwandung zwischen 0,01 und 0,2 insbesondere zwischen 0,02 und 0,1 liegt.

9. Kapsel nach einem der vorangegangenen Ansprüche, **dadurch gekennzeichnet, dass** das thermoplastische Material ausgewählt ist aus der Gruppe bestehend aus Polystyrol, Polyolefinen, insbesondere Polyethylen oder Polypropylen; Polyacrylaten, Polymethacrylaten; Polyimiden, Polycarbonaten, Polyethersulfonen, Polyamiden, Polyestern; und Polyvinylchloriden; Styrol-Butadien-Kautschuk (SBR); Styrol-Ethylen-Butadien-Styrol-Copolymeren (SEBS); Butyl-Kautschuk; Ethylen-Propylen- Kautschuk (EPR); Ethylen-Propylen-Dien-Monomer- Kautschuk (EPDM); Ethylen-Vinylacetat-Copolymer (EVA); Ethylen-Acrylat oder Butadien-Acrylonitril; Maleinsäureanhydridmodifzierten Polymeren und Copolymeren; und gepfropften Copolymeren.

10. Kapsel nach einem der vorangegangenen Ansprüche, **dadurch gekennzeichnet, dass** das Adsorbens ausgewählt ist aus der Gruppe bestehend aus Silicagelen, Zeolithen, trocknenden bzw. feuchtigkeit- oder wasserabsorbierenden Tonen, Alumosilicaten wie Zeolithen oder Bentoniten, Molekularsieben, Aktivkohle, Erdalkalioxiden, Calciumsulfat und deren Gemischen.

11. Kapsel nach einem der vorangegangenen Ansprüche, **dadurch gekennzeichnet, dass** das Adsorbens mindestens ein Entwässerungsmittel ist, vorzugsweise ausgewählt aus Silicagel, Alumosilicaten wie Bentoniten, Molekularsieben und/oder Calciumsulfat.

12. Kapsel nach einem der vorangegangenen Ansprüche, **dadurch gekennzeichnet, dass** die Polymerzusammensetzung bzw. das thermoplastische Material ausgewählt ist aus der Gruppe bestehend aus einem Polymer aus einem einzelnen Monomer, einem Copolymer aus zwei oder mehr Monomeren, einem Gemisch aus zwei oder mehr Polymeren aus je einem einzelnen Monomer, einem Gemisch aus zwei oder mehr Copolymeren, und einem Gemisch aus mindestens einem Polymer aus einem einzelnen Monomer und mindestens einem Copolymer.

13. Kapsel nach einem der vorangegangenen Ansprüche, **dadurch gekennzeichnet, dass** die Polymerzusammensetzung bzw. das thermoplastische Material ein Gemisch darstellt aus mindestens einem Polymer aus einem einzelnen Monomer und mindestens einem Copolymer, vorzugsweise worin mindestens ein Copolymer des thermoplastischen Materials eine gemeinsame Monomereinheit mit dem Polymer aufweist, insbesondere ein Gemisch aus zwei Copolymeren mit mindestens einer gemeinsamen Monomereinheit.

14. Kapsel nach einem der vorangegangenen Ansprüche, **dadurch gekennzeichnet, dass** die Kapselwandung aus der Polymerzusammensetzung mit dem mindestens einen Adsorbens aufweist: mindestens eine Wanderungszone an mindestens einer Oberfläche der Kapselwandung und einen inneren Bereich, worin die maximale Konzentration des Adsorbens innerhalb der Wanderungszone mindestens doppelt so hoch ist wie die maximale Konzentration des Adsorbens in dem inneren Bereich.

15. Kapsel nach einem der vorangegangenen Ansprüche, **dadurch gekennzeichnet, dass** die Kapselwandung aus der Polymerzusammensetzung mit dem mindestens einen Adsorbens homogen aufgebaut ist und eine monolithische Struktur darstellt.

16. Kapsel nach einem der vorangegangenen Ansprüche, **dadurch gekennzeichnet, dass** die Kapselwandung aus zwei oder mehr Schichten aufgebaut ist, insbesondere aus zwei oder drei Schichten.

17. Kapsel nach Anspruch 16, **dadurch gekennzeichnet, dass** die Kapselwandung eine innere und wenigstens einer darüber liegende äußeren Schicht aufweist, wobei die innere Schicht die unmittelbare Wandung des Kapselhohlraums bildet und wobei eine der beiden Schichten aus der Polymerzusammensetzung mit dem mindestens einen Adsorbens und die andere Schicht aus einem pharmakologisch neutralen Material besteht.

18. Kapsel nach Anspruch 16, **dadurch gekennzeichnet, dass** die Kapselwandung eine Sandwichstruktur umfasst, bei der die äußerste Schicht eine Barriere gegen Feuchtigkeit bildet, die mittlere Schicht aus der Polymerzusammensetzung mit dem mindestens einen Adsorbens und die innere Schicht aus einem pharmakologisch neutralen Material besteht.

19. Kapsel nach einem der vorangegangenen Ansprüche, **dadurch gekennzeichnet, dass** die Polymerzusammensetzung Weichmacher, Stabilisatoren, Farbstoffe oder Pigmente enthält.

20. Kapsel nach einem der vorangegangenen Ansprüche, **dadurch gekennzeichnet, dass** die Kapsel eine zweiteilige Kapsel ist, die im geschlossenen Zustand die Form eines Zylinders mit abgerundeten geschlossenen Enden aufweist, der durch einen Kapselkörper, der teleskopartig in eine Kapselkappe eingesteckt wird, gebildet wird.

21. Kapsel nach einem der vorangegangenen Ansprüche, **dadurch gekennzeichnet, dass** die Kapsel aus einem Kapselkörper und einer Kapselkappe besteht, deren Wände 0,1 mm bis 0,5 mm stark sind.

22. Kapsel nach Anspruch 20 oder 21, **dadurch gekennzeichnet, dass** die Nahtstelle zwischen Körper und Kappe durch Verschweißen verschlossen ist.

23. Verwendung einer Polymerzusammensetzung enthaltend mindestens ein Adsorbens zur Herstellung einer Kapsel wie in einem der vorstehenden Ansprüche beschrieben.

24. Inhalator enthaltend mindestens eine Kapsel nach einem der Ansprüche 1 bis 22.

25. Sekundärpackmittel, enthaltend mindestens eine Kapsel nach einem der Ansprüche 1 bis 22.

26. Verfahren zur Trocknung von vordosierten Mengen an inhalationsfähigen Pulvern mit einer Restfeuchte von bis zu 5 Gew.% in Mengen von bis zu 50 mg, bevorzugt bis zu 30 mg, besonders bevorzugt bis zu 15 und ganz besonders bevorzugt bis zu 10 mg, **dadurch gekennzeichnet, dass** das Pulver in einer Kapsel nach Anspruch 1 bis 22 gelagert wird.

27. Verfahren nach Anspruch 26, **dadurch gekennzeichnet, dass** die Kapsel von einem Sekundärpackmittel umgeben ist.

28. Verfahren nach Anspruch 27, **dadurch gekennzeichnet, dass** das Sekundärpackmittel feuchtigkeitsdicht ist.

29. Verfahren nach Anspruch 27 oder 28, **dadurch gekennzeichnet, dass** das Sekundärpackmittel ein Blister ist, der zumindest teilweise eine Aluminiumfolie aufweist.

## Claims

1. Capsule, in particular for packaging inhalation formulations, in which at least one cavity is enclosed by a wall, **characterised in that** at least a part of the wall comprises a polymer composition which contains at least one adsorbent.

2. Capsule according to Claim 1, **characterised in that** it is formed by at least two cylinder-like sub-elements which can be inserted in one another like a telescope, wherein the capsule is an integral component part of a ready-to-use powder inhaler.

3. Capsule according to Claim 1 or 2, **characterised in that** at least a part of the wall consists predominantly, substantially or completely of the polymer composition with at least one adsorbent.

4. Capsule according to any one of the preceding Claims, **characterised in that** the polymer composition contains at least one thermoplastic material.

5. Capsule according to any one of the preceding Claims, **characterised in that** the thermoplastic material comprises at least one polyolefin, particularly preferably polyethylene or polypropylene.

6. Capsule according to any one of the preceding Claims, **characterised in that** the absorbent is present in particle form, in particular with a maximum particle size of less than 50 µm, in particular less than 40 µm, particularly preferably less than 20 µm.

7. Capsule according to any one of the preceding Claims, **characterised in that** the wall thickness of the capsule wall at least in one section thereof lies between approximately 0.05 and 2 mm, in particular between approximately 0.1 and 1.1 mm, more preferably between 0.1 and 0.5 mm.

8. Capsule according to any one of the preceding Claims, **characterised in that** the ratio of the particle size of the absorbent to the wall thickness of the capsule wall lies between 0.01 and 0.2, in particular between 0.02 and 0.1.

9. Capsule according to any one of the preceding Claims, **characterised in that** the thermoplastic material is selected from the group consisting of polystyrene, polyolefins, in particular polyethylene or polypropylene; polyacrylates, polymethyl acrylates, polyimides, polycarbonates, polyether sulphones, polyamides, polyesters; and polyvinyl chlorides; styrene-butadiene rubber (SBR); styrene-ethylene-butadienestyrene copolymers (SEBS); butyl rubber; ethylene-propylene rubber (EPR); ethylene-propylene-diene monomer rubber (EPDM); ethylene-vinyl acetate copolymer (EVA); ethylene acrylate or butadiene acrylonitrile; maleic anhydride modified polymers and copolymers; and graft copolymers.

10. Capsule according to any one of the preceding Claims, **characterised in that** the adsorbent is selected from the group consisting of silica gels, zeolites, drying or moisture- or water-absorbing clays, aluminosilicates such as zeolites or bentonites, molecular sieves, activated carbon, alkaline earth oxides, calcium sulphate and mixtures thereof.

11. Capsule according to any one of the preceding Claims, **characterised in that** the adsorbent is at least one dehydrating agent, preferably selected from silica gel, aluminosilicates such as bentonites, molecular sieves and/or calcium sulphate.

12. Capsule according to any one of the preceding Claims, **characterised in that** the polymer composition or the thermoplastic material is selected from the group consisting of a polymer of a single monomer, a copolymer of two or more monomers, a mixture of two or more polymers in each case of a single monomer, a mixture of two or more copolymers, and a mixture of at least one polymer of a single monomer and at least one copolymer.

13. Capsule according to any one of the preceding Claims, **characterised in that** the polymer composition or the thermoplastic material represents a mixture of at least one polymer of a single monomer and at least one copolymer, preferably wherein at least one copolymer of the thermoplastic material comprises a common monomer unit with the polymer, in particular a mixture of two copolymers with at least one common monomer unit.

14. Capsule according to any one of the preceding Claims, **characterised in that** the capsule wall of the polymer composition with the at least one adsorbent comprises: at least one migration zone at least at one surface of the capsule wall and an inner region, wherein the maximum concentration of the adsorbent within the migration zone is at least twice as high as the maximum concentration of the adsorbent in the inner region.

15. Capsule according to any one of the preceding Claims, **characterised in that** the capsule wall is homogeneously composed of the polymer composition with the at least one adsorbent and represents a monolithic structure.

16. Capsule according to any one of the preceding Claims, **characterised in that** the capsule wall is composed of two or more layers, in particular of two or three layers.

17. Capsule according to Claim 16, **characterised in that** the capsule wall comprises an inner layer and at least one outer layer lying over the latter, wherein the inner layer forms the immediate wall of the capsule cavity, and wherein one of the two layers consists of the polymer composition with the at least one adsorbent and the other layer consists of a pharmacologically neutral material.

18. Capsule according to Claim 16, **characterised in that** the capsule wall comprises a sandwich structure, in which the outermost layer forms a barrier against moisture, the middle layer consists of the polymer composition with the at least one adsorbent and the inner layer consists of a pharmacologically neutral material.

19. Capsule according to any one of the preceding Claims, **characterised in that** the polymer composition contains plasticisers, stabilisers, dyes or pigments.

20. Capsule according to any one of the preceding Claims, **characterised in that** the capsule is a two-part capsule which in the closed state has the form of a cylinder with rounded closed ends which is formed by a capsule body which is inserted in a capsule cap like a telescope.

21. Capsule according to any one of the preceding Claims, **characterised in that** the capsule consists of a capsule body and a capsule cap whose walls are 0.1 mm to 0.5 mm thick.

22. Capsule according to Claim 20 or 21, **characterised in that** the seam between the body and the cap is sealed by welding.

23. Use of a polymer composition containing at least one adsorbent for producing a capsule as described in any one of the preceding Claims.

24. Inhaler containing at least one capsule according to any one of Claims 1 to 22.

25. Secondary packaging means, containing at least one capsule according to any one of Claims 1 to 22.

26. Method for drying pre-dosed quantities of inhalable powders with a residual moisture of up to 5 wt.% in quantities of up to 50 mg, preferably up to 30 mg, particularly preferably up to 15 and most preferably up to 10 mg, **characterised in that** the powder is stored in a capsule according to Claims 1 to 22.

27. Method according to Claim 26, **characterised in that** the capsule is surrounded by a secondary packaging means.

28. Method according to Claim 27, **characterised in that** the secondary packaging means is moisture-tight.

29. Method according to Claim 27 or 28, **characterised in that** the secondary packaging means is a blister pack which at least in part comprises an aluminium foil.

## Revendications

1. Capsule, en particulier pour l'emballage de formulations d'inhalation, pour laquelle au moins une cavité est entourée d'une paroi, **caractérisée en ce qu'**au moins une partie de la paroi présente une composition polymère, laquelle contient au moins un adsorbant.

2. Capsule selon la revendication 1, **caractérisée en ce qu'**elle est formée par au moins deux éléments partiels de type cylindrique, pouvant s'emboîter l'un dans l'autre de manière télescopique, la capsule faisant partie intégrante d'un inhalateur de poudre sèche, prêt à l'emploi.

3. Capsule selon la revendication 1 ou 2, **caractérisée en ce qu'**au moins une partie de la paroi se compose principalement, essentiellement ou entièrement de la composition polymère contenant au moins un adsorbant.

4. Capsule selon l'une quelconque des revendications précédentes, **caractérisée en ce que** la composition polymère contient au moins une matière thermoplastique.

5. Capsule selon l'une quelconque des revendications précédentes, **caractérisée en ce que** la matière thermoplastique comporte au moins une polyoléfine, de manière particulièrement préférée du polyéthylène ou du polypropylène.

6. Capsule selon l'une quelconque des revendications précédentes, **caractérisée en ce que** l'agent d'absorption se présente sous forme de particules, en particulier de particules ayant une taille de particules maximale inférieure à 50 µm, en particulier inférieure à 40 µm, de manière particulièrement préférée inférieure à 20 µm.

7. Capsule selon l'une quelconque des revendications précédentes, **caractérisée en ce que** l'épaisseur de la paroi de la capsule est, au moins dans une partie de la dite paroi, comprise entre environ 0,05 et 2 mm, en particulier entre environ 0,1 et 1,1 mm, de manière particulièrement préférée entre 0,1 et 0,5 mm.

8. Capsule selon l'une quelconque des revendications précédentes, **caractérisée en ce que** le rapport entre la taille de particules de l'agent d'absorption et l'épaisseur de la paroi de la capsule varie entre 0,01 et 0,2, en particulier entre 0,02 et 0, 1 .

9. Capsule selon l'une quelconque des revendications précédentes, **caractérisée en ce que** la matière thermoplastique est choisie dans le groupe constitué de polystyrène, de polyoléfines, en particulier de polyéthylène ou de polypropylène ; de polyacrylates, de polyméthacrylates ; de polyimides, de polycarbonates, de polyéthersulfones, de polyamides, de polyesters ; et de chlorures de polyvinyle ; de caoutchouc au styrène-butadiène (SBR) ; de copolymères styrène-éthylène-butadiène-styrène (SEBS) ; de caoutchouc butyle ; de caoutchouc d'éthylène-propylène (EPR) ; de caoutchouc d'ethylène-propylène-monomère diène (EPDM) ; de copolymère d'ethylène-acétate de vinyle (EVA) ; d'éthylène-acrylate ou de butadiène-acrylonitrile ; de polymères et de copolymères modifiés d'anhydride maléique ; et de copolymères greffés.

10. Capsule selon l'une quelconque des revendications précédentes, **caractérisée en ce que** l'adsorbant est choisi dans le groupe constitué de gels de silice, de zéolithes, d'argiles siccatifs absorbant l'eau ou l'humidité, de silicates d'aluminium comme les zéolithes ou les bentonites, les tamis moléculaires, le charbon actif, les oxydes alcalino-terreux, le sulfate de calcium et leurs mélanges.

11. Capsule selon l'une quelconque des revendications précédentes, **caractérisée en ce que** l'adsorbant est au moins un agent déshydratant, de préférence choisi parmi les gels de silice, les silicates d'aluminium tels que les bentonites, les tamis moléculaires et/ou le sulfate de calcium.

12. Capsule selon l'une quelconque des revendications précédentes, **caractérisée en ce que** la composition polymère et/ou la matière thermoplastique est choisie dans le groupe constitué d'un polymère se composant d'un seul monomère, d'un copolymère se composant de deux monomères ou plus, d'un mélange de deux polymères ou plus respectivement constitués d'un seul monomère, d'un mélange de deux copolymères ou plus, et d'un mélange d'au moins un polymère se composant d'un seul monomère, et d'au moins un copolymère.

13. Capsule selon l'une quelconque des revendications précédentes, **caractérisée en ce que** la composition polymère et/ou la matière thermoplastique représente un mélange d'au moins un polymère constitué d'un seul monomère et d'au moins un copolymère, dans lequel, de préférence, au moins un copolymère de la matière thermoplastique présente un motif monomère commun avec le polymère, en particulier un mélange de deux copolymères comportant au moins un motif monomère commun.

14. Capsule selon l'une quelconque des revendications précédentes, **caractérisée en ce que** la paroi de la capsule constituée de la composition polymère contenant au moins un adsorbant présente : au moins une zone de migration au niveau d'au moins une surface de la paroi de la capsule et une zone intérieure, où la concentration maximale de l'adsorbant à l'intérieur de la zone de migration correspond au moins au double de la concentration maximale de l'adsorbant dans la zone intérieure.

15. Capsule selon l'une quelconque des revendications précédentes, **caractérisée en ce que** la paroi de la capsule constituée de la composition polymère contenant au moins un adsorbant est structurée de manière homogène et représente une structure monolithique.

16. Capsule selon l'une quelconque des revendications précédentes, **caractérisée en ce que** la paroi de la capsule se compose de deux couches ou plus, en particulier de deux ou de trois couches.

17. Capsule selon la revendication 16, **caractérisée en ce que** la paroi de la capsule présente une couche intérieure et au moins une couche extérieure reposant sur cette dernière, la couche intérieure formant la paroi immédiate de la cavité de la capsule et une des deux couches se composant de la composition polymère contenant au moins un adsorbant et l'autre couche se composant d'une matière neutre sur le plan pharmacologique.

18. Capsule selon la revendication 16, **caractérisée en ce que** la paroi de la capsule comporte une structure sandwich, pour laquelle la couche extérieure forme une barrière contre l'humidité, la couche médiane se compose de la composition polymère contenant au moins un adsorbant et la couche intérieure se compose d'une matière neutre sur le plan pharmacologique.

19. Capsule selon l'une quelconque des revendications précédentes, **caractérisée en ce que** la composition polymère contient des plastifiants, des stabilisants, des colorants ou des pigments.

20. Capsule selon l'une quelconque des revendications précédentes, **caractérisée en ce que** la capsule est une capsule en deux parties, laquelle à l'état fermé présente la forme d'un cylindre avec des extrémités fermées arrondies, ledit cylindre étant formé par un corps de capsule qui s'emboîte de manière télescopique dans un chapeau de capsule.

21. Capsule selon l'une quelconque des revendications précédentes, **caractérisée en ce que** la capsule se compose d'un corps de capsule et d'un chapeau de capsule, dont les parois ont une épaisseur allant de 0,1 mm à 0,5 mm.

22. Capsule selon la revendication 20 ou 21, **caractérisée en ce que** le joint entre le corps et le chapeau est fermé par soudage.

23. Utilisation d'une composition polymère contenant au moins un adsorbant pour la fabrication d'une capsule telle que décrite des les revendications précédentes.

24. Inhalateur contenant au moins une capsule selon l'une quelconque des revendications 1 à 22.

25. Emballage secondaire contenant au moins une capsule selon l'une quelconque des revendications 1 à 22.

26. Procédé de dessiccation de quantités préalablement dosées de poudres pouvant être inhalées, avec une humidité résiduelle allant jusqu'à 5 % en poids dans des quantités pouvant atteindre 50 mg maximum, de préférence jusqu'à 30 mg, de manière particulièrement préférée jusqu'à 15 et de manière tout particulièrement préférée jusqu'à 10 mg, **caractérisé en ce que** la poudre est stockée dans une capsule selon l'une quelconque des revendications 1 à 22.

27. Procédé selon la revendication 26, **caractérisé en ce que** la capsule est entourée d'un emballage secondaire.

28. Procédé selon la revendication 27, **caractérisé en ce que** l'emballage secondaire est étanche à l'humidité.

29. Procédé selon la revendication 27 ou 28, **caractérisé en ce que** l'emballage secondaire est un blister, qui présente au moins partiellement une feuille d'aluminium.
